# EUROPEAN PATENT SPECIFICATION

(11) **EP 3 049 145 B1**
(45) Date of publication and mention of the grant of the patent: **17.06.2020**
(21) Application number: 14833364.4
(22) Date of filing: 25.09.2014
(51) Int. Cl.: A61K 9/00, A61B 5/00, A61K 9/48, A61M 31/00

(54) **DELIVERY CAPSULE WITH THRESHOLD RELEASE**
ABGABEKAPSEL MIT SCHWELLENFREISETZUNG
CAPSULE D'ADMINISTRATION À LIBÉRATION SEUIL

(30) Priority: 26.09.2013 US 201361882965 P
(43) Date of publication of application: 03.08.2016
(60) Divisional application: 20174752.4
(73) Proprietor: Progenity, Inc., San Diego, CA 92122 (US)
(72) Inventor: ZOU, Hans, Chappaqua, NY 10514 (US); SHIMIZU, Jeffrey, Cortlandt Manor, NY 10567 (US); IORDANOV, Ventzeslav, NL-5551 TZ Valkenswaard (NL); KERKHOT, Klaas, NL-5672 RH Nuenen (NL); WANKE, Christoph, NL-5504 EW Veldhoven (NL)
(74) Representative: Mewburn Ellis LLP
(86) International application number: PCT/IB2014/002848
(87) International publication number: WO 2015/059569

(56) References cited:
- WO-A1-94/01165
- WO-A2-2004/066903
- WO-A2-2010/142284
- GB-A- 2 178 958

## Description

### CROSS-REFERENCE TO RELATED APPLICATIONS

The present application is an International Application claiming priority to U.S. Provisional Patent Application No. 61/882,965 filed September 26, 2013.

### BACKGROUND

Electronic capsules have been proposed for controlling the delivery of a substance in a mammal's body. In one use, a swallowable capsule carries the substance in a sealed reservoir and includes an electro-mechanical drive mechanism that forces the drug from the reservoir, through an opening in the capsule, and into the surrounding gastrointestinal tract.
WO 2004066903 A2 discloses capsules comprising a housing, a compressible reservoir configured to obtain a substance to be dispensed from the capsule, an opening in the housing providing an outlet for the substance contained in the reservoir, and a threshold valve sealing the opening until a pressure in the capsule exceeds the threshold pressure.
WO 2010142284 A2 discloses a capsule with an opening in its housing providing an outlet of a substance and a threshold valve sealing the housing, until a pressure in the capsule exceeds a threshold pressure. A gas generating unit is placed within a polyethylene bag which expands, thereby pushing the substance out of the capsule.
WO 9401165 A1 discloses a housing, a gas generating unit, a compressible reservoir, an opening in the housing and a threshold valve sealing the opening, until a pressure in the capsule exceeds the threshold pressure.
GB 2 178 958 A discloses a capsule comprising a housing, a gas generating unit, a compressible reservoir sealed relative to the gas generating unit via a diaphragm and an opening in the housing. The gas is generated via the reaction of a fluid which enters the capsule via the semi-permeable wall, thereby forming gas pushing out the respective substance.

### BRIEF DESCRIPTION OF THE DRAWINGS

The detailed description is described with reference to the accompanying figures. In the figures, the left-most digit(s) of a reference number identifies the figure in which the reference number first appears. The use of the same reference numbers in different figures indicates similar or identical components or features.

### SUMMARY OF INVENTION

The invention provides a capsule as defined in the appended claims.
FIG. 1A illustrates, in cross-section, a capsule including a payload compartment including a deformable portion and a gas generating unit according to one implementation of this disclosure.
FIG. 1B illustrates the capsule of FIG. 1A after the contents of the payload compartment have been released from the capsule.
FIG. 2 illustrates, in cross-section, a capsule including a payload compartment and a gas-generating unit according to another implementation of this disclosure.
FIG. 3 illustrates, in cross-section, a capsule including a payload compartment and a plurality of gas-generating units according to another implementation of this disclosure.
FIG. 4 illustrates, in cross-section, a capsule including a plurality of reservoirs, each having a corresponding threshold release valve and gas generating unit.
FIG. 5 illustrates, in cross-section, a capsule including a payload compartment and a platen acted upon by gas generated by a gas generating unit to dispense the contents of the payload compartment.

### DETAILED DESCRIPTION

This disclosure describes a capsule, such as an ingestible capsule, configured for insertion into a mammalian body to release a substance in that body. The capsule generally includes a payload compartment, or reservoir, that contains the substance. An opening is formed in the capsule to provide an egress through which the substance can exit the capsule, and a threshold valve is provided in the opening. The threshold valve retains the substance in the reservoir until a pressure in the reservoir reaches a threshold pressure, at which time the threshold valve opens to release some or all of the substance through the opening. In some implementations, the threshold valve is a single use valve whereas in other embodiments it is a re-sealable valve that allows for iterative opening and closing, to control an amount and/or timing of release of the substance. A gas generating unit is provided in the capsule, to generate a gas that provides an internal pressure in the capsule, which eventually creates the threshold pressure that expels the substance through the threshold valve.

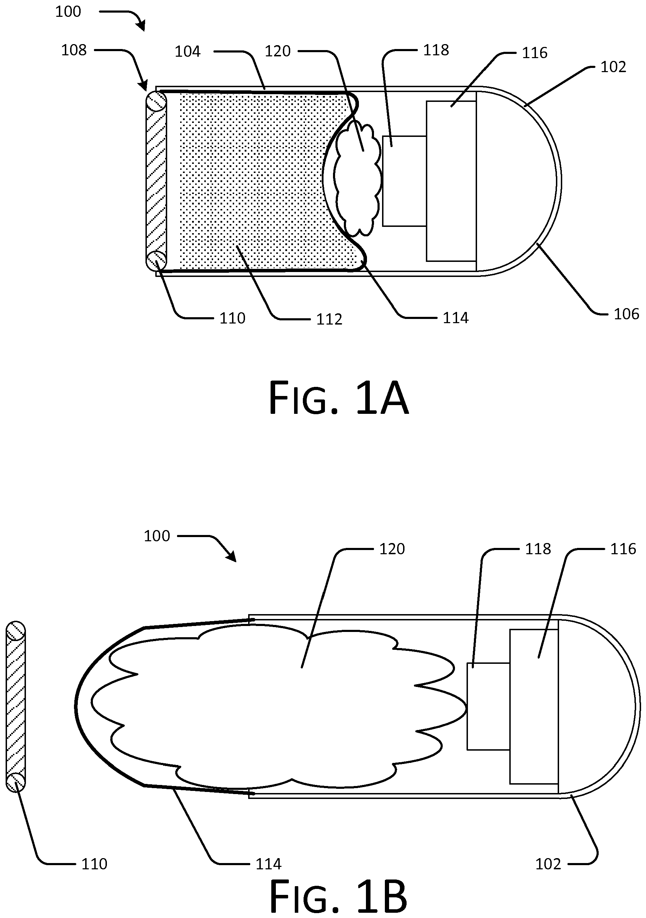

FIG. 1 illustrates a capsule 100 according to one embodiment of this disclosure. The capsule 100 generally includes a housing 102 shaped for placement in a mammalian body through an orifice in that body. For example, the capsule may be sized and shaped to be swallowed by a mammal. In other implementations, the capsule may be designed for insertion into a different body orifice. For example, the capsule may be intended for insertion into a woman's vaginal canal or uterus. In such an embodiment, the capsule may longer and/or thinner than the capsule illustrated. Moreover, a capsule for insertion may also be formed on or otherwise attached to a carrier structure that facilitates insertion of the capsule. In still other implementation, the capsule may be constructed as an implant, and placed subcutaneously. Because the capsule is intended for placement in a mammalian body, the capsule preferably is formed of a non-degrading, biocompatible material. By way of non-limiting example, the capsule may be formed from a biocompatible plastic or stainless steel.

As illustrated in FIG. 1A, the housing 102 includes a substantially cylindrical sidewall 104 and a rounded end 106. An opening 108 is formed at an axial end of the sidewall 104, opposite the rounded end 106. The shape of the housing is not limited to the illustrated configuration; the housing may take any configuration that allows for insertion into the mammalian body.

In the embodiment illustrated in FIG. 1A, the opening 108 is sealed with a threshold valve 110. As will be described in more detail below, the threshold valve 110 preferably seals the opening 108 until the pressure inside the capsule reaches a threshold pressure that opens the threshold valve, thereby allowing contents of the capsule to exit through the opening. In the illustrated embodiment, the threshold valve 110 is a disc-shaped member that creates a seal with an inner surface of the sidewall 104, proximate the opening 108. In some embodiments, the threshold valve 110 may be a sealing gasket that seals the opening 108, forming a closed end of the capsule 100. In other embodiments, the threshold valve 110 may comprise more than one piece, such as a relatively hard disc surrounded by an O-ring or similar sealing structure.

A reservoir 112 is disposed in the housing, in fluid communication with the opening 108, for storing the contents to be released from the capsule. In the illustrated embodiment, the reservoir 112 is a sealed space, bounded by the threshold valve 110 and a deformable membrane 114. The membrane 114 may be fixed to an inner wall of the housing 102, proximate the opening 108. In other embodiments, a portion of the sidewall 104 may also define at least a portion of the reservoir 112. As will be appreciated, in the illustration of FIG. 1A, removing, or opening, the threshold valve 110 will allow for the contents of the reservoir to exit the capsule through the opening 108.

Also illustrated schematically in FIG. 1A, the capsule 100 includes electronics 116 and a gas generating unit 118. The electronics 116 and the gas generating unit 118 preferably are retained in the capsule such that they do not come into contact with the substance contained in the reservoir. In FIG. 1A the substance to be administered is sealed in the reservoir 112, and thus sealed relative to the electronics 116 and the gas generating unit 118. The electronics 116 and the gas generating unit 118 may also be sealed relative to each other.

The electronics 116 may be any number of electronic components that contribute to the operation and functionality of the capsule 100. For example, the electronics 116 may include a power source such as a battery, logic circuitry, one or more sensors, an antenna, a receiver, a transmitter, a transceiver, control electronics, and/or the like. The electronics 116 may vary depending upon the design of the capsule, as will be appreciated from this disclosure.

The gas generating unit 118 is a mechanism that selectively generates and/or releases a gas. In some embodiments, the generating unit 118 is of a type including an electrolytic cell filled with an aqueous solution and that has at least two electrodes. When a voltage is applied across the electrodes, hydrogen and oxygen gas are generated, which gasses are, in turn, released from the gas generating unit 118.

In operation, as the gas generating unit 118 generates a gas 120, the gas 120 expands in the capsule 100. Continued gas generation leads to an increased pressure inside the capsule 100, which pressure eventually becomes sufficient that it acts on the deformable membrane 114. In Figure 1A, the gas 120 is illustrated as filling a space between the gas generating unit and the membrane 114, to press the membrane 114 toward the opening 108. In some embodiments, a separate chamber may be provided in which the gas is generated, while in others the gas is free to expand anywhere in the capsule. Continued pressure on the deformable membrane 114 applies a pressure to the reservoir 112, which increasing pressure, in turn, applies a pressure on the threshold valve 110. When the increasing internal pressure in the reservoir reaches a threshold pressure, the threshold valve opens, releasing the contents of the reservoir 112 through the opening 108.

Figure 1B illustrates the capsule 100 of FIG. 1A after enough of the gas 120 has been generated that the threshold pressure has been reached, and the threshold valve 120 has been opened. In this embodiment, the threshold pressure opens the threshold valve 110 by completely dislodging the valve 110 from the housing 102. More specifically, the valve 110 was retained in place by a frictional or interference fit with the sidewall 104, and the force maintaining that seal was overcome, thus dislodging the valve 110. Choice of materials used for the valve 110 and the sidewall 104 will dictate the threshold pressure in these embodiments.

Also in FIG. 1B, a portion of the deformable membrane 114 has been pushed through the opening 108. More specifically, the deformable membrane 114 is still attached to the sidewall 104 in FIG. 1B, but has been turned "inside-out" from its original position. In other embodiments the deformable membrane may not extend through the opening in this manner, but such a construction may result in a more complete release of the substance contained in the reservoir, i.e., because the substance is actively pushed out of the capsule, into the surrounding environment. Moreover, when the inner surface of the reservoir is exposed to the environment, as in FIG. 1B, constituents in the environment, e.g., gastro-intestinal fluid, may assist in removing the contents from that surface.

Although the threshold valve 110 is shown as being completely removed from the housing 102 in FIG. 1B, in other embodiments the threshold valve 110 may remain attached to the housing 102 after the valve becomes dislodged from the housing. For example, the threshold valve 110 may be hinged or tethered, e.g., to the sidewall 104, of the housing 102. Such arrangements may promote easier recovery of the complete capsule.

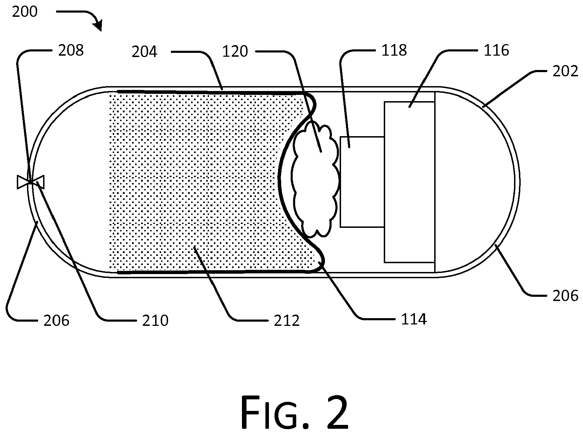

FIG. 2 illustrates another example embodiment of a capsule 200. In FIG. 2, the capsule 200 includes a housing 202 having a cylindrical sidewall 204 terminating at two rounded ends 206. An opening 208 is formed through one of the ends 206. The opening 208 provides a fluid passageway between a reservoir 212 and the exterior of the housing 202.

In operation, as in the embodiments described in connection with FIGs. 1A and 1B, the gas generating unit 118 of FIG. 2 generates the gas 120 that expands and presses on the deformable membrane 114. As the membrane deforms, the pressure inside the reservoir increases, eventually to a threshold pressure sufficient to open the threshold valve 210, allowing the contents of the reservoir 212 to leave the capsule 200, through the opening 208. Unlike in the previous embodiments, however, as the contents of the reservoir 212 exit and the pressure inside the reservoir decreases, the pressure will eventually drop below a re-sealing pressure, and the threshold valve 210 will reseal. The re-sealing pressure may be the same as or lower than the threshold pressure, depending upon the design of the threshold valve 210. Moreover, once the threshold valve is re-sealed, with continued gas generation, the pressure may again increase to the threshold pressure, to again discharge contents from the reservoir. As will be appreciated, the capsule 100 illustrated in FIGs. 1A and 1B provides a one-time, or bolus, release of the contents of the reservoir, whereas the embodiment of FIG. 2 may allow for the contents to be released all at once, or in several, smaller releases.

In one implementation of the capsule 200, the gas 120 may be continuously generated. In this implementation, the threshold valve 210 opens each time the pressure in the capsule builds to the threshold pressure, and then closes as the substance is released and the pressure drops back to the re-sealing pressure. Because the gas continues to be generated, the pressure will again build to the threshold pressure, and the process of releasing and re-sealing will repeat. As will be appreciated, design and construction of the threshold valve 210 and the gas generating unit 118 will dictate how much substance is released, and how frequently it is released. Thus, a release profile may be varied, depending upon the gas generation unit and/or the threshold valve used.

In other implementations of the capsule 200, release of the gas 120 may be controlled, e.g., selectively started and stopped, to provide a desired release profile of the substance. For example, in some embodiments, the gas generation may be stopped when the threshold valve 210 opens, such that the valve closes upon a reduction in pressure to the re-sealing pressure, and remains closed until the gas generation is re-started, and the gas again reaches the threshold pressure. In one embodiment, once the threshold valve opens, or shortly thereafter, application of the voltage across the electrodes in the gas generating unit is stopped, and thus gas generation is stopped. At a later time, the voltage may be reapplied across the electrodes, to again begin gas generation, eventually resulting in another dispensing of the contents of the reservoir.

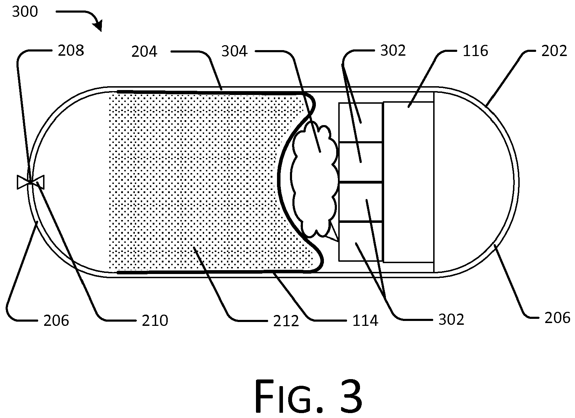

Another embodiment of a capsule 300 is illustrated in FIG. 3. The capsule 300 is similar in most aspects to the capsule 200 illustrated in FIG. 2, but the capsule 300 includes a plurality of gas generating units 302. Although four gas generating units 302 are illustrated, more or fewer may alternatively be provided. In some uses of the capsule 300, each of the gas generating units 302 may be capable of releasing a gas 304 sufficient to open the threshold valve 210. Thus, some amount of the substance contained in the reservoir 212 will be released each time one of the gas generating units 302 is triggered. For example, one of the gas generating units 302 may be triggered, such that a pressure reaches the threshold pressure and the contents are expelled. Such expulsion will cause a decrease in the internal pressure of the capsule, until the threshold valve 210 re-seals. Then, at an appropriate, perhaps pre-determined time, another of the gas generating units 302 may be triggered, resulting in a release of additional contents from the reservoir.

In the embodiment of FIG. 3, the amount of content released from the reservoir when each of the gas generating units 302 is triggered will be dictated in part by the amount of gas released by each unit 302 and in part by the specifics of the threshold valve 210. Moreover, the speed at which each of the gas generating units 302 generates the gas 304 will impact how quickly the contents are expelled from the capsule, i.e., relative to the time at which the respective gas generating unit 302 is triggered.

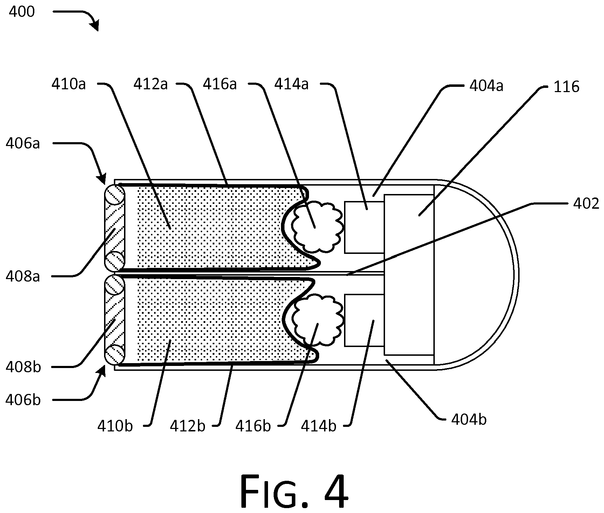

Although in the implementation of FIG. 3 the triggering of each of the gas generating units 302 will result in expulsion of some amount of the contents of the reservoir 212, in another embodiment, each gas generating unit may be associated with a separate reservoir. An example of such an embodiment is illustrated in FIG. 4.

In FIG. 4, a capsule 400 includes an internal partition 402 that helps to define a plurality of internal compartments 404a, 404b. In some embodiments, the compartments 404a, 404b are sealed relative to each other. Although two compartments 404a, 404b are illustrated in FIG. 4, more compartments may be formed inside the capsule.

Each compartment 404a, 404b includes an opening 406a, 406b, and as illustrated, each opening is sealed with a threshold valve 408a, 408b. In the illustrated embodiment the threshold valves 408a, 408b are similar in construction to the threshold valve 110 of the embodiment of FIG. 1A, although the threshold valves may instead be similar in construction to the threshold valve 210 illustrated in FIGs. 2 and 3. Each of the compartments 404a, 404b has a respective reservoir 410a, 410b disposed therein. As in previously-described embodiments, the reservoirs 410a, 410b, may include a deformable membrane 412a, 412b.

Gas generating units 414a, 414b also are provided in the capsule 400, one corresponding to each reservoir 410a, 410b. As with previous embodiments, the gas generating units 414a, 414b, when activated, produce a gas 416a, 416b. The generated gas 416a, 416b, deforms the respective membranes 412a, 412b, applying a pressure to the respective threshold valves 408a, 408b. As in the embodiment of FIGs. 1A and 1B, as the pressure in the respective compartments reaches and exceeds a threshold pressure, the threshold valves 408a, 408b open, allowing the contents of the reservoirs 410a, 410b to be released from the capsule 400.

The embodiment of FIG. 4 may provide for a multiple release arrangement that is more easily controlled than the use of the threshold valve 210 that opens and re-seals. Moreover, the capsule 400 may allow for administration of more than one substance, i.e., by containing a different substance in each of the reservoirs 410a, 410b.

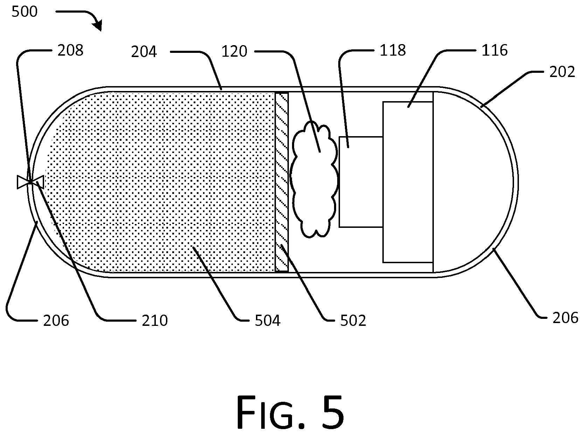

Another capsule 500 is illustrated in FIG. 5. The capsule 500 is similar in construction to the capsule 200 described above and illustrated in FIG. 2, but the capsule 500 includes a platen 502 movable axially in the capsule 500. In the illustrated embodiment, the platen 502 serves to define a portion of a reservoir 504 containing a substance for dispensing. The platen 502 is illustrated schematically as being movable relative to and along the sidewall 204. In some embodiments, the platen 502 and/or the inner surface of the sidewall 204 may include features that assist or otherwise guide this movement. For example, the platen may have features on its periphery that key the platen 502 to the sidewall 204. Such features may prevent rotation of the platen 502 relative to the sidewall 204. In another embodiment, a shaft may be provided in the capsule, arranged parallel to a longitudinal axis of the capsule, and the platen includes a cutout sized to receive the shaft. In such an embodiment, the platen 502 rides along the shaft. The platen 502 may be keyed to the shaft, to prevent rotation of the platen relative to the capsule.

In the capsule 500, the reservoir 504 is preferably sealed, such that contents of the reservoir 504 remain in the reservoir until being released through the opening 208. To seal the reservoir 504, the platen 502 may be sealed relative to the sidewall 204. For example, a wiper seal or the like may be provided on the periphery of the platen 502 for contacting the sidewall 204. Other seals are also known, and may be used to seal the reservoir 504. Other methods may also be used to seal the reservoir. For example, a deformable membrane such as one of those illustrated in previous embodiments may also be used in the embodiment of FIG. 5. In such an arrangement, the generated gas 120 moves the platen 502, which acts on the deformable membrane to increase the pressure in the reservoir. It may be beneficial that in this embodiment the platen need not be sealed relative to the sidewall.

As described throughout this disclosure, operation of each of the example capsules is preferably controlled and/or performed using the electronics 116, which are illustrated schematically in each of the Figures.

In embodiments of this disclosure, the electronics 116 preferably are constructed such that they control the generation of gas by the gas generating unit(s) and thus the delivery of the substance(s) contained in the capsule. The electronics 116 may be pre-programmed, i.e., programmed before swallowing or insertion. For example, in the embodiment illustrated in FIG. 4, the control electronics 116 may be pre-programmed to time the release of each of the reservoirs 410a, 410b. The pre-programming may include an instruction to trigger gas release at a certain time, e.g., measured from the time of swallowing or an activation of the capsule, or when a certain condition is met, such as a sensed condition in the body, such as a pH-level, a temperature, or the like. To this end, the control electronics 116 may include one or more sensors, such as a pH sensor and/or a thermometer. Other or different sensors may also be included.

The functioning of the capsules may also be partly or completely dictated from outside the body to which the capsule has been administered. For example, the control electronics 116 may include a receiver that receives instructions, such as from outside the body or from a sensor associated with the capsule. For instance, an administrator may track the position of the capsule in the mammal, such as through a global positioning or other positioning sensor included in the electronics 116 and instruct gas generation, as appropriate, for example, via a wireless transmission.

As should be appreciated, the electronics 116 may enable capsules according to embodiments of this disclosure to provide targeted administration of substances, for example, at a specific location and/or a specific time. Such a targeted administration is useful in clinical studies, e.g., to determine efficacy of a drug-in-test at various locations along the GI tract, and in administration, e.g., to ensure a drug is administered where and/or when it will be most effective.

Embodiments of the disclosure may also be well suited for administering different types of products. For example, the embodiments illustrated in FIGs. 1A, 1B, and 4, in which the threshold valve is a single-use valve, may be better suited for delivering solid, granular, or powdered substances, whereas the valves illustrated in FIGs. 2, 3, and 5 may be better suited for delivery of liquid or suspended-formulations. In addition, as noted above in the discussion of FIG. 4, the capsule 400 that has a plurality of reservoirs 410a, 410b, may be used to administer several different drugs via the same capsule. Of course, use of disclosed embodiments is not intended to be limited to delivering any formulation.

The illustrated embodiments are provided as examples, and modifications to the embodiments may be appreciated by those having ordinary skill in the art with the benefit of this disclosure. By way of non-limiting example, different types of threshold valves may be used than those illustrated specifically in the Figures. For example, the multi-use threshold valve of FIG. 2 may be used in conjunction with some or all of the multiple reservoirs shown in FIG. 4.

Moreover, although FIG. 4 illustrates multiple reservoirs, each containing an amount of a substance to be dispensed into the mammalian body, one reservoir may contain multiple substances. For example, a single reservoir may be filled with a plurality of different types of substances, e.g., drugs, to be dispensed to the mammal. Alternatively, a single reservoir may contain multiple compartments. The compartments may be maintained by a partition or the like, which may be a membrane disposed or otherwise formed in the reservoir. In some embodiments each partitioned compartment is in fluid communication with the opening, such that all compartments are evacuated through the opening. In other embodiments, the partition may be rupturable, such that the multiple partitions mix or otherwise come into fluid communication with each other, before the contents of the reservoir are dispensed. The rupturable membrane may be chosen to rupture at a pressure below the threshold pressure, while in other embodiments the membrane may be mechanically ruptured by a rupturing member disposed in the reservoir.

In each of the illustrated embodiments, the capsule is a complete capsule, i.e., in that it includes both the payload element (including the reservoir) and the drive element (including the gas generating unit). In some embodiments, the payload element and the drive element may be formed separately, and joined at a later time, for example, at the time of administration. In this manner, different payload elements, i.e., having different contents or amounts, may be used, as necessary for each patient. The payload element and the drive element may be formed as separate halves of a complete capsule, for example, that are mated together to form the complete capsule. They may be mated together, for example, using a threaded engagement, a snap-fit engagement, or the like.

Although the subject matter has been described in language specific to structural features, it is to be understood that the subject matter defined in the appended claims is not necessarily limited to the specific features described. Rather, the specific features are disclosed as illustrative forms of implementing the claims.

## Claims

1. A capsule (100; 200; 300; 400; 500) comprising:
a housing (102; 202);
a gas generating unit (118; 302) for generating gas (120; 304) in the housing (102; 202);
a compressible reservoir (112; 212) sealed relative to the gas generating unit (118; 302) and configured to obtain a substance to be dispensed from the capsule;
an opening (108; 208) in the housing (102; 202) providing an outlet for the substance contained in the reservoir (112; 202); and
a threshold valve (110; 210) sealing the opening (108; 208) until a pressure in the capsule (100; 200; 300; 400; 500) exceeds a threshold pressure,
a deformable membrane (114) forming at least a portion of the compressible reservoir (112; 212),
**characterized in that**:
the deformable membrane (114) is configured to extend outside the housing (102; 202) when the threshold valve (110; 210) opens.

2. The capsule (100; 200; 300; 400; 500) of claim 1, wherein the threshold valve (110; 210) dislodges from the housing (102; 202) when the pressure in the capsule (100; 200; 300; 400;500) exceeds the threshold pressure.

3. The capsule (100; 200; 300; 400; 500) of claim 1, wherein the deformable membrane (114) is attached to an inner wall of the housing (102; 202) proximate the opening (108; 208).

4. The capsule (100; 200; 300; 400; 500) of claim 1, further comprising an additional one or more gas generating units (118; 302) for generating gas (120; 304) in the housing (102; 202).

5. The capsule (100; 200; 300; 400; 500) of the-claim 1, further comprising a platen (502) movable relative to the housing (102; 202) to compress the reservoir (112; 212).

6. The capsule (100; 200; 300; 400; 500) of claim 5, wherein the platen (502) is sealed relative to the housing (102; 202).

7. The capsule (100; 200; 300; 400; 500) of claim 1, wherein the substance is one of a solid, a powder, a fluid, or a suspension.

8. The capsule (100; 200; 300; 400; 500) of claim 1, wherein the gas generating unit (118; 302) comprises an electrolytic cell.

9. The capsule (100; 200; 300; 400; 500) of claim 1, wherein the gas generating unit (118; 302) further comprises a plurality of electrodes.

10. The capsule (100; 200; 300; 400; 500) of claim 1, further comprising control electronics (116) for instructing the gas generating unit (118; 302) to generate gas (120; 304).

11. The capsule (100; 200; 300; 400; 500) of claim 10, wherein the control electronics (116) for instructing the gas generating unit (118; 302) to generate gas (120; 304) include a dosage schedule including information about at least one of a time or a condition to generate gas (120; 304).

## Patentansprüche

1. Kapsel (100; 200; 300; 400; 500), umfassend:
ein Gehäuse (102; 202);
eine Gaserzeugungseinheit (118; 302) zum Erzeugen von Gas (120; 304) in dem Gehäuse (102; 202);
ein komprimierbares Reservoir (112; 212), das relativ zu der Gaserzeugungseinheit (118; 302) abgedichtet ist und ausgelegt ist, um eine Substanz zum Abgeben von der Kapsel zu erhalten;
eine Öffnung (108; 208) in dem Gehäuse (102; 202), die einen Auslass für die in dem Reservoir (112; 202) enthaltene Substanz bereitstellt; und
ein Schwellwertventil (110; 210), das die Öffnung (108; 208) abdichtet, bis ein Druck in der Kapsel (100; 200; 300; 400; 500) einen Druckschwellwert überschreitet,
eine verformbare Membran (114), die zumindest einen Abschnitt des komprimierbaren Reservoirs (112; 212) ausbildet,
**dadurch gekennzeichnet, dass**:
die verformbare Membran (114) ausgelegt ist, um sich aus dem Gehäuse (102; 202) hinaus zu erstrecken, wenn sich das Schwellwertventil (110; 210) öffnet.

2. Kapsel (100; 200; 300; 400; 500) nach Anspruch 1, wobei das Schwellwertventil (110; 210) sich von dem Gehäuse (102; 202) wegbewegt, wenn der Druck in der Kapsel (100; 200; 300; 400; 500) den Druckschwellwert überschreitet.

3. Kapsel (100; 200; 300; 400; 500) nach Anspruch 1, wobei die verformbare Membran (114) mit einer Innenwand des Gehäuses (102; 202) in der Nähe der Öffnung (108; 208) verbunden ist.

4. Kapsel (100; 200; 300; 400; 500) nach Anspruch 1, ferner umfassend eine zusätzliche oder mehrere zusätzliche Gaserzeugungseinheiten (118; 302) zum Erzeugen von Gas (120; 304) in dem Gehäuse (102; 202).

5. Kapsel (100; 200; 300; 400; 500) nach Anspruch 1, ferner umfassend eine Platte (502), die relativ zu dem Gehäuse (102; 202) beweglich ist, um das Reservoir (112; 212) zu komprimieren.

6. Kapsel (100; 200; 300; 400; 500) nach Anspruch 5, wobei die Platte (502) relativ zu dem Gehäuse (102; 202) abgedichtet ist.

7. Kapsel (100; 200; 300; 400; 500) nach Anspruch 1, wobei die Substanz eine aus einem Feststoff, einem Pulver, einer Flüssigkeit oder einer Suspension ist.

8. Kapsel (100; 200; 300; 400; 500) nach Anspruch 1, wobei die Gaserzeugungseinheit (118; 302) eine Elektrolysezelle umfasst.

9. Kapsel (100; 200; 300; 400; 500) nach Anspruch 1, wobei die Gaserzeugungseinheit (118; 302) ferner eine Vielzahl von Elektroden umfasst.

10. Kapsel (100; 200; 300; 400; 500) nach Anspruch 1, ferner umfassend eine Steuerelektronik (116), um die Gaserzeugungseinheit (118; 302) zum Erzeugen von Gas (120; 304) anzuweisen.

11. Kapsel (100; 200; 300; 400; 500) nach Anspruch 10, wobei die Steuerelektronik (116), die die Gaserzeugungseinheit (118; 302) zum Erzeugen von Gas (120; 304) anweist, ein Dosierungsschema umfasst, das Informationen über zumindest einen aus einem Zeitpunkt oder einem Zustand zum Erzeugen von Gas (120; 304) umfasst.

## Revendications

1. Capsule (100 ; 200 ; 300 ; 400 ; 500) comprenant :
un boîtier (102 ; 202) ;
une unité de génération de gaz (118 ; 302) pour générer du gaz (120 ; 304) dans le boîtier (102 ; 202) ;
un réservoir compressible (112 ; 212) scellé par rapport à l'unité de génération de gaz (118 ; 302) et configuré pour obtenir une substance devant être distribuée à partir de la capsule ;
une ouverture (108 ; 208) dans le boîtier (102 ; 202) fournissant une sortie pour la substance contenue dans le réservoir (112 ; 202) ; et
une vanne à seuil (110 ; 210) scellant l'ouverture (108 ; 208) jusqu'à ce qu'une pression dans la capsule (100 ; 200 ; 300 ; 400 ; 500) dépasse une pression de seuil,
une membrane déformable (114) formant au moins une partie du réservoir compressible (112 ; 212),
**caractérisé en ce que** :
la membrane déformable (114) est configurée pour s'étendre à l'extérieur du boîtier (102 ; 202) lorsque la vanne de seuil (110 ; 210) s'ouvre.

2. Capsule (100 ; 200 ; 300 ; 400 ; 500) selon la revendication 1, dans laquelle la vanne à seuil (110 ; 210) est délogée du boîtier (102 ; 202) lorsque la pression dans la capsule (100 ; 200 ; 300 ; 400 ; 500) dépasse la pression de seuil.

3. Capsule (100 ; 200 ; 300 ; 400 ; 500) selon la revendication 1, dans laquelle la membrane déformable (114) est fixée à une paroi intérieure du boîtier (102 ; 202) à proximité de l'ouverture (108 ; 208).

4. Capsule (100 ; 200 ; 300 ; 400 ; 500) selon la revendication 1, comprenant en outre une ou plusieurs unités de génération de gaz supplémentaires (118 ; 302) pour générer du gaz (120 ; 304) dans le boîtier (102 ; 202).

5. Capsule (100 ; 200 ; 300 ; 400 ; 500) selon la revendication 1, comprenant en outre une platine (502) mobile par rapport au boîtier (102 ; 202) pour comprimer le réservoir (112 ; 212).

6. Capsule (100 ; 200 ; 300 ; 400 ; 500) selon la revendication 5, dans laquelle la platine (502) est scellée par rapport au boîtier (102 ; 202).

7. Capsule (100 ; 200 ; 300 ; 400 ; 500) selon la revendication 1, dans laquelle la substance est un parmi un solide, une poudre, un fluide ou une suspension.

8. Capsule (100 ; 200 ; 300 ; 400 ; 500) selon la revendication 1, dans laquelle l'unité de génération de gaz (118 ; 302) comprend une cellule électrolytique.

9. Capsule (100 ; 200 ; 300 ; 400 ; 500) selon la revendication 1, dans laquelle l'unité de génération de gaz (118 ; 302) comprend en outre une pluralité d'électrodes.

10. Capsule (100 ; 200 ; 300 ; 400 ; 500) selon la revendication 1, comprenant en outre une électronique de commande (116) pour ordonner à l'unité de génération de gaz (118 ; 302) de générer du gaz (120 ; 304).

11. Capsule (100 ; 200 ; 300 ; 400 ; 500) selon la revendication 10, dans laquelle l'électronique de commande (116) pour ordonner à l'unité de génération de gaz (118 ; 302) de générer du gaz (120 ; 304) comprend un programme de dosage comprenant des informations sur au moins un parmi un temps ou une condition pour générer du gaz (120 ; 304).
